# EUROPEAN PATENT SPECIFICATION

(11) **EP 3 153 498 B1**
(45) Date of publication and mention of the grant of the patent: **21.08.2019**
(21) Application number: 15803170.8
(22) Date of filing: 06.01.2015
(51) Int. Cl.: C07C 255/58, C07C 253/30, C07D 271/07

(54) **N-SUBSTITUTED PHENYL GLYCINE PREPARATION METHOD**
VERFAHREN ZUR HERSTELLUNG VON N-SUBSTITUIERTEM PHENYLGLYCIN
PROCÉDÉ DE PRÉPARATION DE PHÉNYL GLYCINE N-SUBSTITUTÉE

(30) Priority: 05.06.2014 CN 201410245765
(43) Date of publication of application: 12.04.2017
(73) Proprietor: ABA Chemicals Corporation, Taicang City, Jiangsu 215433 (CN)
(72) Inventor: LIN, Zhigang, Taicang Jiangsu 215433 (CN); LIU, Yanqin, Taicang Jiangsu 215433 (CN); XU, Jun, Taicang Jiangsu 215433 (CN); JIANG, Yueheng, Taicang Jiangsu 215433 (CN); CAI, Tong, Taicang Jiangsu 215433 (CN)
(74) Representative: Proi World Intellectual Property GmbH
(86) International application number: PCT/CN2015/070181
(87) International publication number: WO 2015/184798

(56) References cited:
- EP-A1- 0 911 333
- CN-A- 1 142 223
- CN-A- 1 861 596
- CN-A- 101 921 203
- CN-A- 103 992 241
- ABBOTT P A ET AL: "Fused mesoionic heterocycles: synthesis of [1,2,3]triazolo[1,5-a]quinoline, [1,2,3]triazolo[1,5-a]quinazoline, [1,2,3]triazolo[1,5-a]quinoxaline and [1,2,3]triazolo[5,1-c]benzotriazine derivatives", TETRAHEDRON, vol. 58, no. 16, 15 April 2002 (2002-04-15), pages 3185-3198, XP004347818, ISSN: 0040-4020, DOI: 10.1016/S0040-4020(02)00269-7
- BROEDERS, FABIENNE ET AL: "Practical Syntheses of Oxindole Derivatives: Chemical Development towards 2-(5-Chloro-2-oxo-2,3-dihydroindol-1-yl)ac etamide and (S)-2-(5-Chloro-2-oxo-2,3-dihydroindol-1-y l)propionamide", ORGANIC PROCESS RESEARCH & DEVELOPMENT, vol. 13, no. 3, 30 January 2009 (2009-01-30), pages 442-449, XP002776975, ISSN: 1083-6160, DOI: 10.1021/OP800203P

## Description

### Technical Field

The Invention relates to a method for preparing N-substituted-phenyl glycine (a Dabigatran etexilate intermediate).

### Background Art

N-substituted-phenyl glycine is an important intermediate of Dabigatran Etexilate, a new anticoagulant developed and marketed by Boehringer Ingelheim Company of Germany. The synthetic method provided by the published reports (WO2012009678, WO2009111997, CN1861596 and US20050107355, etc.) is as follows:

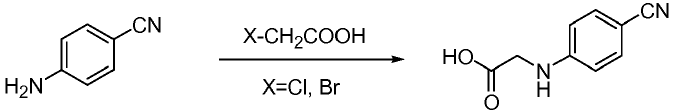

With this method, if chloroacetic acid is used as the raw material, the product will have a poor purity and a low yield; if bromoacetic acid is used as the raw material, the cost will be high.

EP0911333 relates to compounds capable of exhibiting inhibition of a phosphodiesterase (PDE) enzyme.

"Fused mesoionic heterocycles: synthesis of [1,2,3]triazolo[1,5-a]quinoline, [1,2,3]triazolo[1,5-a]quinazoline, [1,2,3]triazolo[1,5-a]quinoxaline and [1,2,3]triazolo[5,1-c]benzotriazine derivatives" by ABBOTT P A ET AL, published by TETRAHEDRON, ELSEVIER SCIENCE PUBLISHERS, AMSTERDAM, NL, vol. 58, no. 16, 15 April 2002, pages 3185 - 3198, discusses general method for the synthesis of mesoionic derivatives of [1,2,3]triazolo[1,5-a]quinoline, [1,2,3]triazolo[1,5-a]quinazoline, [1,2,3]triazolo[1,5-a]quinoxaline and [1,2,3]triazolo[5,1-c]benzotriazine.

### Summary of the Invention

The technical problem to be solved by the Invention is to provide a new method for preparing N-substituted-phenyl glycine (a Dabigatran etexilate intermediate).

The technical scheme adopted by the invention is provided in independent claim 1. Various improvements of the scheme are recited in the dependent claims.

The above preparation method is expressed by a chemical equation as follows:

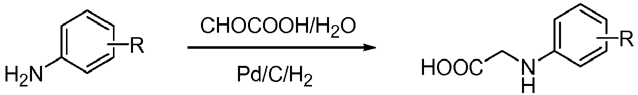

The beneficial effects of the Invention are that: the synthetic route has not been reported ever before; moreover, with cheap and widely available raw materials, simple unit operation and low requirement for equipment, the method is suitable for industrialized mass production. The product in the Invention has high purity and yield.

The partial additional aspects and advantages of the Invention are given as follows, some of which will be obvious through the following description, or be understood in practice of the invention.

### Detailed Description of the Preferred Embodiments

The following detailed embodiments are further descriptions of the technical schemes of the Invention, but the Invention is not limited to this.

### (1) Synthesis of N-cyano phenyl glycine (I)

Add 45.9g of 50%(w/w) glyoxalic acid water solution, 500ml of methyl alcohol, 0.5g of palladium-carbon with 5% palladium load and 35.4g of 4-cyanoaniline into a 1000 mL hydrogenation reaction cauldron for stirring under ambient temperature (18-25°C) for 30 minutes; conduct hydrogen substitution in the system (it refers to substituting the air in the reaction cauldron with hydrogen and maintain a pressure of 10 atm (wherein 1 atm = 101325 Pa) this is a professional control process for production; the pressure cannot be increased to 10 atm (wherein 1 atm = 101325 Pa) at the beginning as the pressure increases with the temperature rise and the heat emission by the reaction. Generally, supplement 2-3 atm (wherein 1 atm = 101325 Pa) hydrogen firstly; after the temperature rises to 50°C and gets stable, increase hydrogen to 10 atm (wherein 1 atm = 101325 Pa); all of these processes are controlled by DSC control system of a PC), slowly raise the temperature to 50°C for reaction; maintain a pressure of 10 atm (wherein 1 atm = 101325 Pa) for reaction for 12h; control the raw material below 0.2% through HPLC monitoring; generate a esterified product with a mass ratio of 20% (the reactive solvent is alcohol, therefore a corresponding esterified impurities will be generated. But the impurities can be easily hydrolyzed with alkali and returned to the target product acid). (The later steps are to hydrolyze impurities and return them to the target compounds); cool it to ambient temperature and filter it to recycle the palladium-carbon (palladium is a precious metal, therefore, the palladium-carbon will always been recycled after use). Filter the solution and reduce the pressure for concentration; add 120ml of 10%(W/W) sodium hydroxide solution into the residue; raise the temperature to 50°C for reaction for 1 hour; add 10%(W/W) hydrochloric acid to adjust the pH value to 3 (through the former alkalization treatment, the product salt will be dissolved in water; therefore, it shall be acidized and dissolved out as a solid.); cool it to 0°C, filter it and wash it with water to obtain N-cyano phenyl glycine and obtain 49.2g of product through vacuum drying; calculated yield: 93%; purity: 99.5%; LC-MS(m/e):176.1. The chemical equation is as follows:

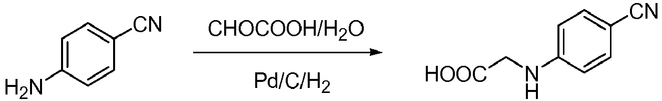

### (2) Synthesis of N-cyano phenyl glycine (II)

Add 45.9g of 50% glyoxalic acid water solution, 500ml of THF, 0.5g of palladium-carbon with 5% palladium load and 35.4g of 4-cyanoaniline into a 1000 mL hydrogenation reaction cauldron for stirring under ambient temperature for 30 minutes; conduct hydrogen substitution in the system; slowly raise the temperature to 50°C for reaction; maintain a pressure of 10atm for reaction for 12h; control the raw material below 0.2% through HPLC monitoring. Cool it to ambient temperature and filter it to recycle the palladium-carbon. Filter the solution and reduce the pressure for concentration; add 100ml of water into the residue; raise the temperature to 50°C for reaction; add 10%(W/W) hydrochloric acid to adjust the pH value to 3; cool it to 0°C, filter it and wash it with water to obtain N-cyano phenyl glycine and obtain 50.2g of product through vacuum drying; calculated yield: 94.9%; purity: 99.4%; LC-MS(m/e):176.1.

### (3) Synthesis of N-cyano phenyl glycine (III)

Add 45.9g of 50% glyoxalic acid water solution, 500ml of methyl alcohol, 0.5g of palladium-carbon with 5% palladium load, 16g of sodium carbonate and 53.1g of 4-cyanoaniline into a 1000 mL hydrogenation reaction cauldron for stirring under ambient temperature for 30 minutes; conduct hydrogen substitution in the system; slowly raise the temperature to 50°C for reaction; maintain a pressure of 10atm for reaction for 12h; control the raw material below 0.2% through HPLC monitoring. Cool it to ambient temperature and filter it to recycle the palladium-carbon. Filter the solution and reduce the pressure for concentration; add 100ml of water into the residue; raise the temperature to 50°C for reaction; add 10%(W/W) hydrochloric acid to adjust the pH value to 3; cool it to 0°C, filter it and wash it with water to obtain N-cyano phenyl glycine and obtain 50.4g of product through vacuum drying; calculated yield: 95.2%; purity: 99.4%; LC-MS(m/e):176.1.

### (4) Synthesis of N-4-[(2H)-1,2,4-oxadiazole-5-ketone-3-yl] phenyl glycine (I)

Add 45.9g of 50% glyoxalic acid water solution, 500ml of methyl alcohol, 0.5g of palladium-carbon with 5% palladium load and 53.1g of 3-(4-aminophenyl)-(2H)-1,2,4-oxadiazole-5-ketone into a 1000 mL hydrogenation reaction cauldron for stirring under ambient temperature for 30 minutes; conduct hydrogen substitution in the system; slowly raise the temperature to 50°C for reaction; maintain a pressure of 10atm for reaction for 12h; control the raw material below 0.2% through HPLC monitoring; generate a esterified product with a mass ratio of 20%. Cool it to ambient temperature and filter it to recycle the palladium-carbon. Filter the solution and reduce the pressure for concentration; add 120ml of 10%(W/W) sodium hydroxide solution into the residue; raise the temperature to 50°C for reaction for 1 hour; add 10%(W/W) hydrochloric acid to adjust the pH value to 3; cool it to 0°C, filter it and wash it with water to obtain N-4-[(2H)-1,2,4-oxadiazole-5-ketone-3-yl] phenyl glycine and obtain 66.5g of product through vacuum drying; calculated yield: 94.2%; purity: 99.5%; LC-MS(m/e):235.1.

The chemical equation is as follows:

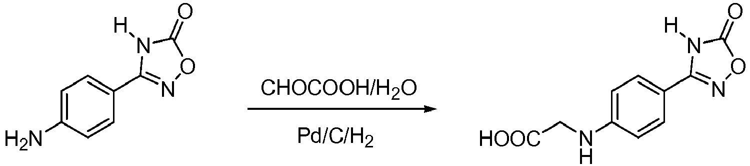

### (5) Synthesis of N-4-[(2H)-1,2,4-oxadiazole-5-ketone-3-yl] phenyl glycine (II)

Add 45.9g of 50% glyoxalic acid water solution, 500ml of THF, 0.5g of palladium-carbon with 5% palladium load and 53.1g of 3-(4-aminophenyl)-(2H)-1,2,4-oxadiazole-5-ketone into a 1000 mL hydrogenation reaction cauldron for stirring under ambient temperature for 30 minutes; conduct hydrogen substitution in the system; slowly raise the temperature to 50°C for reaction; maintain a pressure of 10atm for reaction for 12h; control the raw material below 0.2% through HPLC monitoring. Cool it to ambient temperature and filter it to recycle the palladium-carbon. Filter the solution and reduce the pressure for concentration; add 100ml of water into the residue; raise the temperature to 50°C for reaction; add 10%(W/W) hydrochloric acid to adjust the pH value to 3; cool it to 0°C, filter it and wash it with water to obtain N-4-[(2H)-1,2,4-oxadiazole-5-ketone-3-yl] phenyl glycine and obtain 66g of product through vacuum drying; calculated yield: 93.5%; purity: 99.6%; LC-MS(m/e):176.1.

### (6) Synthesis of N-4-[(2H)-1,2,4-oxadiazole-5-ketone-3-yl] phenyl glycine (III)

Add 45.9g of 50% glyoxalic acid water solution, 500ml of methyl alcohol, 0.5g of palladium-carbon with 5% palladium load, 16g of sodium carbonate and 53.1g of 3-(4-aminophenyl)-(2H)-1,2,4-oxadiazole-5-ketone into a 1000 mL hydrogenation reaction cauldron for stirring under ambient temperature for 30 minutes; conduct hydrogen substitution in the system; slowly raise the temperature to 50°C for reaction; maintain a pressure of 10atm for reaction for 12h; control the raw material below 0.2% through HPLC monitoring. Cool it to ambient temperature and filter it to recycle the palladium-carbon. Filter the solution and reduce the pressure for concentration; add 100ml of water into the residue; raise the temperature to 50°C for reaction; add 10%(W/W) hydrochloric acid to adjust the pH value to 3; cool it to 0°C, filter it and wash it with water to obtain N-4-[(2H)-1,2,4-oxadiazole-5-ketone-3-yl] phenyl glycine and obtain 67g of product through vacuum drying; calculated yield: 95%; purity: 99.3%; LC-MS(m/e):176.1.

## Claims

1. A method for preparing N-substituted-phenyl glycine, wherein, it comprises the following steps:
(1) taking substituted-aniline as the raw material to form an imine intermediate with glyoxylic acid; and
(2) said imine intermediate generates N-substituted-phenyl glycine through reduction reaction under the action of palladium-carbon/hydrogen;
**characterized in that** in Step (2) said palladium-carbon is with a palladium load of 5% (w/w).

2. The preparation method according to Claim 1, wherein, in Step (1), the mole ratio between glyoxylic acid and the raw material substituted-aniline is (1-2):1.

3. The preparation method according to Claim 1, wherein, in Step (2), the said reduction reaction occurs in a water/organic solvent system, wherein, the organic solvent is the solvent mixed with one, two or more of methyl alcohol, methyl alcohol, propyl alcohol, THF and dioxane.

4. The preparation method according to Claim 1, wherein, in Step (2), the mole ratio between the said palladium-carbon and the raw material substituted-aniline is (0.01-1):1.

5. The preparation method according to Claim 4, wherein, in Step (2), the temperature for the said reaction is 0-100°C and the time is 1-24 hours.

6. The preparation method according to Claim 5, wherein, in Step (2), the temperature for the said reaction is 45-55°C and the time is 10-14 hours.

7. The preparation method according to Claim 1, wherein, in Step (2), the system is also added with sodium carbonate, potassium carbonate or sodium hydroxide with the equal mole ratio of the substituted-aniline.

8. The preparation method according to Claim 1, wherein, the said N-substituted-phenyl glycine is N-cyano phenyl glycine or N-4-[(2H)-1,2,4-oxadiazole-5-ketone-3-yl] phenyl glycine.

## Patentansprüche

1. Verfahren zur Herstellung von N-substituiertem Phenylglycin, welches folgende Schritte umfasst:
(1) Nehmen von substituiertem Anilin als Ausgangsmaterial zur Bildung eines Imin-Zwischenprodukts mit Glyoxylsäure; und
(2) das Imin-Zwischenprodukt erzeugt durch Reduktionsreaktion unter Einwirkung von Palladium-Kohlenstoff/Wasserstoff N-substituiertes Phenylglycin.

2. Herstellungsverfahren nach Anspruch 1, wobei in Schritt (1) das Molverhältnis zwischen Glyoxylsäure und dem substituierten Rohmaterial-Anilin (1-2):1 beträgt.

3. Herstellungsverfahren nach Anspruch 1, wobei in Schritt (2) die Reduktionsreaktion in einem Wasser-/organischen Lösungsmittelsystem stattfindet, wobei das organische Lösungsmittel das Lösungsmittel ist, das mit einem, zwei oder mehreren von Methylalkohol, Methylalkohol, Propylalkohol, THF und Dioxan gemischt ist.

4. Herstellungsverfahren nach Anspruch 1, wobei in Schritt (2) das Molverhältnis zwischen dem Palladium-Kohlenstoff und dem substituierten Rohmaterial-Anilin (0,01-1):1 beträgt.

5. Herstellungsverfahren nach Anspruch 4, wobei in Schritt (2) die Temperatur für die Reaktion 0 bis 100 °C und die Zeit 1 bis 24 Stunden beträgt.

6. Herstellungsverfahren nach Anspruch 5, wobei in Schritt (2) die Temperatur für die Reaktion 45 bis 55 °C beträgt und die Zeit 10 bis 14 Stunden beträgt.

7. Herstellungsverfahren nach Anspruch 1, wobei in Schritt (2) dem System auch Natriumcarbonat, Kaliumcarbonat oder Natriumhydroxid mit dem gleichen Molverhältnis des substituierten Anilins zugesetzt wird.

8. Herstellungsverfahren nach Anspruch 1, wobei das N-substituierte Phenylglycin N-Cyanophenylglycin oder N-4 -[(2H)-1,2,4-oxadiazol-5-keton-3-yl]-Phenylglycin ist.

## Revendications

1. Procédé de préparation de phénylglycine N-substituée, le procédé comprenant les étapes suivantes :
(1) prendre de l'aniline substituée comme matière première pour former un imine intermédiaire avec l'acide glyoxylique ; et
(2) ledit intermédiaire imine génère de la phénylglycine N-substituée par réaction de réduction sous l'action du palladium-carbone/hydrogène ;
**caractérisé en ce que** dans l'étape (2), ledit palladium-carbone présente une charge en palladium de 5 % (p/p).

2. Procédé de préparation selon la revendication 1, dans lequel, à l'étape (1), le rapport molaire entre l'acide glyoxylique et la matière première aniline substituée est (1-2):1.

3. Procédé de préparation selon la revendication 1, dans lequel, à l'étape (2), ladite réaction de réduction se produit dans un système eau/solvant organique, dans lequel le solvant organique est le solvant mélangé avec un, deux ou plus parmi l'alcool méthylique, l'alcool méthylique, l'alcool propylique, le THF et le dioxane.

4. Procédé de préparation selon la revendication 1, dans lequel, à l'étape (2), le rapport molaire entre ledit palladium-carbone et la matière première aniline substituée est (0,01-1):1.

5. Procédé de préparation selon la revendication 4, dans lequel, à l'étape (2), la température destinée à ladite réaction est de 0 à 100 °C et le temps est de 1 à 24 heures.

6. Procédé de préparation selon la revendication 5, dans lequel, à l'étape (2), la température destinée à ladite réaction est de 45 à 55 °C et le temps est de 10 à 14 heures.

7. Procédé de préparation selon la revendication 1, dans lequel, à l'étape (2), on ajoute également au système du carbonate de sodium, du carbonate de potassium ou de l'hydroxyde de sodium avec le même rapport molaire que l'aniline substituée.

8. Procédé de préparation selon la revendication 1, dans lequel ladite phénylglycine N-substituée est la N-cyano phénylglycine ou la N-4-[(2H)-1,2,4-oxadiazole-5-cétone-3-yl]phénylglycine.
